# FASCICULE DE BREVET EUROPEEN

(11) **EP 2 978 836 B1**
(45) Date de publication et mention de la délivrance du brevet: **26.09.2018**
(21) Numéro de dépôt: 14713106.4
(22) Date de dépôt: 27.03.2014
(51) Int. Cl.: C12N 1/12, C12P 19/42, C12P 23/00, C12P 7/64, C12P 17/18

(54) **PROCÉDÉ DE STABILISATION DES MÉTABOLITES SENSIBLES À L'OXYDATION PRODUITS PAR LES MICROALGUES DU GENRE CHLORELLA**
VERFAHREN ZUR STABILISIERUNG OXIDATIONSEMPFINDLICHER METABOLITEN DURCH MIKROALGEN DER GATTUNG CHLORELLA
METHOD FOR STABILISING OXIDATION-SENSITIVE METABOLITES PRODUCED BY MICROALGAE OF THE CHLORELLA GENUS

(30) Priorité: 29.03.2013 FR 1352856
(43) Date de publication de la demande: 03.02.2016
(73) Titulaire: Roquette Frères, 62136 Lestrem (FR)
(72) Inventeur: MACQUART, Gabriel, F-62350 Mont Bernanchon (FR)
(74) Mandataire: Cabinet Plasseraud
(86) Numéro de dépôt international: PCT/EP2014/056122
(87) Numéro de publication internationale: WO 2014/154785

(56) Documents cités:
- US-A1- 2010 303 989
- SANSAWA H ET AL: "Production of intracellular phytochemicals in Chlorella under heterotrophic conditions", JOURNAL OF BIOSCIENCE AND BIOENGINEERING, ELSEVIER, AMSTERDAM, NL, vol. 98, no. 6, 1 janvier 2004 (2004-01-01), pages 437-444, XP004727088, ISSN: 1389-1723
- DOUCHA JIRI ET AL: "Production of high-density Chlorella culture grown in fermenters", JOURNAL OF APPLIED PHYCOLOGY, vol. 24, no. 1, janvier 2012 (2012-01), pages 35-43, XP002717867, ISSN: 0921-8971
- WANG Y ET AL: "Growth-associated biosynthesis of astaxanthin in heterotrophic Chlorella zofingiensis (Chlorophyta)", WORLD JOURNAL OF MICROBIOLOGY AND BIOTECHNOLOGY, KLUWER ACADEMIC PUBLISHERS, DO, vol. 24, no. 9, 22 février 2008 (2008-02-22), pages 1915-1922, XP019617114, ISSN: 1573-0972
- ZHENG-YUN WU ET AL: "Modeling of lutein production by heterotrophic Chlorella in batch and fed-batch cultures", WORLD JOURNAL OF MICROBIOLOGY AND BIOTECHNOLOGY, KLUWER ACADEMIC PUBLISHERS, DO, vol. 23, no. 9, 6 février 2007 (2007-02-06), pages 1233-1238, XP019535141, ISSN: 1573-0972, DOI: 10.1007/S11274-007-9354-2
- CHEN F ET AL: "EFFECT OF CARBON TO NITROGEN RATIO AND AERATION ON THE FATTY ACID COMPOSITION OF HETEROTROPHIC CHLORELLA-SOROKINIANA", JOURNAL OF APPLIED PHYCOLOGY, KLUWER, DORDRECHT, NL, vol. 3, no. 3, 1 septembre 1991 (1991-09-01), pages 203-210, XP008166059, ISSN: 0921-8971, DOI: 10.1007/BF00003578
- SHI X M ET AL: "High-yield production of lutein by the green microalga Chlorella protothecoides in heterotrophic fed-batch culture", BIOTECHNOLOGY PROGRESS, AMERICAN INSTITUTE OF CHEMICAL ENGINEERS, US, vol. 18, no. 4, 1 July 2002 (2002-07-01), pages 723-727, XP002689085, ISSN: 8756-7938, DOI: 10.1021/BP0101987 [retrieved on 2002-07-17]

## Description

La présente invention est relative à un procédé de stabilisation des métabolites sensibles à l'oxydation produits par des microalgues, plus particulièrement du genre *Chlorella.*

Parmi les métabolites sensibles à l'oxydation les plus connus, on trouve par exemple les caroténoïdes.

Les caroténoïdes sont des pigments plutôt orange et jaunes répandus chez de très nombreux organismes vivants. Liposolubles, ils sont en général facilement assimilables par les organismes.

Ils appartiennent à la famille chimique des terpénoïdes, formés à partir de la polymérisation d'unités isoprènes à structure aliphatique ou alicyclique. Il est généralement admis qu'ils suivent des voies métaboliques similaires à celles des lipides.

Ils sont synthétisés par toutes les plantes vertes et par de nombreux champignons, bactéries (dont les cyanobactéries) et par toutes les microalgues.

Ils sont absorbés par les animaux dans leur nourriture.

Les caroténoïdes ont été étudiés pour la prévention du cancer et d'autres maladies humaines, car ils présentent des propriétés antioxydantes remarquables.

Ce sont d'ailleurs leurs propriétés antioxydantes qui rendent les caroténoïdes particulièrement sensibles à l'oxydation, car il est bien connu de l'homme du métier qu'une substance antioxydante doit être elle-même facilement oxydable pour jouer ce rôle.

Des exemples représentatifs de caroténoïdes comprennent l'α-carotène, le β-carotène et le lycopène.

Le β-carotène et le lycopène sont généralement présents sous une forme non combinée libre, qui est emprisonnée au sein des chloroplastes des cellules végétales.

Les xanthophylles sont des molécules de couleur jaune dérivées des carotènes par ajout d'atomes d'oxygène (fonctions alcool, cétone, époxy, ...). Elles font partie de la famille de caroténoïdes.

Les xanthophylles sont abondantes dans un certain nombre de fruits jaunes ou oranges et légumes tels que les pêches, les mangues, la papaye, les pruneaux, les courges et les oranges.

On les rencontre également dans les chloroplastes ou les chromoplastes des cellules végétales, notamment dans les pétales de certaines fleurs de couleur jaune, orange ou rouge, et chez les algues, par exemple les algues brunes (Phéophycées), où elles masquent la chlorophylle.

Les xanthophylles sont des antioxydants qui contribuent entre autres à la santé des yeux.

Des exemples de xanthophylles comprennent la lutéine, l'astaxanthine, la canthaxanthine, la zéaxanthine, la cryptoxanthine...

La forme libre des caroténoïdes permet une meilleure absorption lorsqu'ils sont consommés dans les aliments ou comme complément alimentaire.

La lutéine est un pigment xanthophylle de formule 4[18-(4-hydroxy-2,6,6-triméthylcyclohex-1-én-I-yl)-3,7,12,16-tetraméthyloctadéca-1,3,5,7,9,11,13,15,17-nonaén-I-yl]-3,5,5-triméthylcyclohex-2-én-I-ol trouvé en concentrations élevées dans la macula de l'oeil et dans la partie centrale de la rétine.

Elle y joue un rôle important dans la filtration des longueurs d'onde ultraviolettes de la lumière afin d'éviter d'endommager la lentille de l'oeil et la macula.

La lutéine a par ailleurs des propriétés antioxydantes qui permettent également de protéger la macula, qui est riche en acides gras polyinsaturés, des radicaux libres induits par la lumière.

La lutéine ne peut pas être produite par le corps et, par conséquent, doit être apportée par l'alimentation.

Ainsi, la lutéine est devenue de plus en plus utilisée dans les compléments nutritionnels pour la prévention et / ou le traitement des pertes de vision dues à la dégénérescence maculaire liée à l'âge (ou DMLA), les cataractes, et la rétinite pigmentaire.

Les microalgues de type *Muriellopsis* sp., *Scenedesmus almeriensis, Chlorella zofingiensis, Chlorella sorokiniana* et *Chlorella protothecoides* ont déjà été proposées comme des sources potentielles de lutéine.

Du point de vue réglementaire, la lutéine est obtenue par extraction au solvant de souches de fruits et de végétaux comestibles ainsi que des herbes, de la luzerne et de *Tagetes erecta.*

Différentes quantités de carotènes peuvent également être présentes.

La lutéine peut contenir des matières grasses et cires naturellement présentes dans le matériel végétal d'origine.

Seuls les solvants suivants sont autorisés pour leur extraction: méthanol, éthanol, propanol-2, hexane, acétone, méthyléthylcétone et anhydride carbonique.

Certaines préparations commerciales de lutéine sont vendues comme ayant « 5 % ou 10 % de lutéine ». Ces préparations sont en fait de la lutéine purifiée (estérifiée ou libre) ajoutée à un agent stabilisant inerte dans une proportion de 5 à 15% pour assurer sa stabilisation.

Sensible à la lumière et à l'oxydation, elle doit donc être stockée dans un contenant hermétiquement fermé, résistant à la lumière et à l'oxygène, dans un endroit frais et sec. Et malgré ces précautions, la stabilité de ces composés n'est pas totalement assurée.

Ces conditions de stockage et de manipulation ne sont en outre guère aisées.

La lutéine se rencontrant en forte proportion dans certaines microalgues comme *Chlorella,* il est donc préféré de choisir cette source microbienne afin de développer des procédés de production permettant d'obtenir de plus grandes quantités de lutéine de façon rentable et compenser la perte inhérente à la fragilité de telles molécules.

Par ailleurs, les microalgues du genre *Chlorella* renferment une gamme unique de composants qui comprend, outre les caroténoïdes précités, tous les acides aminés essentiels, des acides gras saturés et insaturés, une grande quantité de vitamines, minéraux et oligo-éléments, ainsi que des composants précieux, dont la chlorophylle, en forte concentration.

Les microalgues sont également une source de nourriture potentielle attrayante riche en protéines et autres nutriments essentiels ; une fois sèches, elles représentent classiquement un apport d'environ 45 % de protéines, 20 % de matières grasses, 20 % de glucides, 5 % de fibres et 10 % de minéraux et vitamines.

Cependant, au même titre que les caroténoïdes, les acides gras insaturés présentent une sensibilité d'autant plus élevée à l'oxydation que leur degré d'insaturation est élevé ; l'acide linoléique polyinsaturé est ainsi plus sensible à l'oxydation que les acides monoinsaturés de type oléique et palmitoléique.

Il en est de même pour les pigments chlorophylliens, dont la sensibilité à la photo-oxydation est bien connue de l'Homme du métier.

Il est ainsi connu que les sels solubles de la chlorophylle ont une activité antioxydante plus de 1000 fois supérieure à celle des xanthines, et 20 fois supérieure à celle du resvératrol (polyphénol de la classe des stilbènes présent dans certains fruits comme les raisins, les mûres...).

Les suppléments de chlorophylle se retrouvent ainsi dans le commerce sous forme liquide, en comprimés ou en capsules. La chlorophylle est souvent incluse dans les formules d'aliments verts en poudre.

Quant aux vitamines, les vitamines B (vitamine B9 ou vitamine B12, par exemple), hydrosolubles, elles sont naturellement fragiles (sensibilité à la lumière, à la chaleur et à l'oxydation).

L'importance des vitamines A, C et E comme antioxydants dans la biochimie des organismes vivants est également bien documentée.

Pour augmenter la résistance de tous ces éléments nutritifs, il est donc nécessaire de les protéger des « agressions extérieures », i.e. lumière et oxygène.

Or les voies classiques de préparation de ces composés passent par leur extraction / purification de leur milieu biologique de base, puis leur cloisonnement dans des récipients hermétiques.

Des additifs antioxydants et des conditions de stockage sous atmosphère inerte sont donc préconisés. Mais, outre le fait que ces technologies sont complexes et couteuses à mettre en oeuvre, elles ne sont pas très efficaces ni totalement satisfaisante au point de vue nutritionnel et santé.

Une solution technique alternative a été présentée, notamment dans le brevet US 2005/0186298, consistant à stabiliser des caroténoïdes dans la biomasse des microalgues qui les produisent.

Il s'agit plus particulièrement ici de stabiliser la biomasse de *Haemotococcus pluvialis* productrice d'astaxanthine.

Cependant, cette solution technique recommande :
- de produire la biomasse sèche (classiquement réalisée en conditions phototrophiques - croissance en présence de lumière et de CO₂ - mais pouvant être produite également en hétérotrophie - fermentation à l'obscurité en présence d'une source carbonée assimilable), et ensuite
- de combiner cette biomasse avec un mélange d'au minimum deux antioxydants de type tocophérols.

Par conséquent, le problème sous-jacent de la présente invention est de proposer un procédé alternatif de stabilisation des métabolites sensibles à l'oxydation, et plus particulièrement ceux produits par des microalgues, notamment celles du genre *Chlorella,* par un procédé simple, sans qu'il ne soit utile d'ajouter des substances chimiques de type antioxydants ou stabilisants.

Soucieuse de mettre au point un procédé plus efficace que ceux connus de l'état de l'art, la société Demanderesse a développé ses propres recherches et a réussi à adapter les technologies de production des microalgues en hétérotrophie pour parvenir à cette fin.

La culture des chlorophytes, et plus particulièrement de *Chlorella,* par voie hétérotrophique est connue de manière générale pour la préparation de biomasses riches en métabolites d'intérêt, dont la lutéine.

Il est ainsi admis depuis les années 1960 qu'il est même possible d'obtenir des rendements en pigments beaucoup plus importants qu'avec les mêmes microalgues cultivées plus classiquement en conditions auxotrophiques éclairées.

Le choix de cette voie de production hétérotrophique, comme énoncé ci-avant, a surtout pour objectif de produire les teneurs en lutéine les plus hautes possibles, permettant ainsi de compenser leur perte par dégradation oxydative.

Shi et al (2002, Biotechnoloy Progress, American Institute of Chemical Engineers, vol 18, 723-727) décrit la production de lutéine par culture fermentaire hétérotrophique en mode discontinu par des microalgues du genre *Chlorella* protothecoides. Sansawa et al (2004, J Bioscience and Bioengineering, 98, 437-444), Doucha et al (2012, J Applied Phycology, 24, 35-43) et Wang et al (2008, World J. Microbiol. Biotechnol 24, 1915-1922) décrivent des procédés de fermentation d'une biomasse de microalgues du genre *Chlorella* comprenant une phase de culture carencée en glucose. Wu et al, dans leur article de 2007 publié dans la revue World J. Microbiol. Biotechnol., volume 23, pp 1233 - 1238, ont ainsi modélisé la production de lutéine par culture fermentaire hétérotrophique en mode discontinu (type « batch ») et semi continu (type « fed-batch ») afin d'optimiser la production de lutéine. US2010/0303989 divulgue un procédé de production d'huile par Chlorella protothecoides comprenant la fermentation de Chlorella en conditions hétérotrophiques comprenant une étape de fermentation en mode "fed batch". La concentration de glucose a été surveillée. Lorsque la concentration en glucose était faible, plus de glucose était ajouté au fermenteur.

Cependant, aucun des documents de l'état de l'art, à la connaissance de la société Demanderesse, ne décrit ni ne suggère d'utiliser la biomasse elle-même, produite en conditions hétérotrophiques, comme vecteur de stabilisation des métabolites d'intérêt.

Bien au contraire, il est recommandé d'ajouter des antioxydants pour parvenir à ce résultat.

La présente invention porte sur un procédé tel que décrit dans les revendications.

La présente description décrit un procédé de stabilisation ou de stockage de métabolites sensibles à l'oxydation choisis dans le groupe constitué des caroténoïdes comme la lutéine, des acides gras monoinsaturés et polyinsaturés comme l'acide palmitoléique, l'acide oléique ou l'acide linoléique, des pigments chlorophylliens comme la chlorophylle A et B, et des vitamines comme les vitamines B9 et B12, pris seuls ou en combinaison, plus particulièrement des caroténoïdes, le dit procédé comprenant :
- la fermentation d'une biomasse de microalgues en conditions hétérotrophiques comprenant une phase de culture carencée en un facteur nutritif ; et
- le stockage de la biomasse sèche au sein de laquelle les métabolites sensibles à l'oxydation sont stabilisés.

Par « stabilisation des métabolites », on entend garantir la qualité des métabolites d'intérêt après stockage pendant une période de plus d'un an, ce qui se traduit notamment par une protection des métabolites contre leur dégradation oxydative. Notamment, le stockage peut être réalisé à température ambiante sous atmosphère non inerte. L'étape de stockage dure au moins 12, 18 ou 24 mois, de préférence à température ambiante.

Par « métabolites d'intérêt sensibles à l'oxydation » produits par les microalgues du genre *Chlorella,* on entend des composés choisis dans le groupe constitué des caroténoïdes incluant la lutéine, des acides gras monoinsaturés et polyinsaturés incluant l'acide palmitoléique, l'acide oléique et l'acide linoléique, des pigments chlorophylliens tels que la chlorophylle A et B, et des vitamines, incluant la vitamine B9 et notamment la vitamine B12, plus particulièrement des caroténoïdes.

Par phase de culture « carencée en un facteur nutritif » on entend une phase de culture dans laquelle au moins un des facteurs nutritifs de la microalgue est apportée en quantité insuffisante pour permettre sa croissance normale. Il est à noter que par quantité insuffisante n'est pas entendu un apport nul en ce facteur nutritif. Cette phase de carence nutritive conduit alors à ralentir (limiter) le métabolisme cellulaire, sans l'inhiber totalement.

La culture est par exemple réalisée dans des conditions telles que l'un des facteurs nutritifs est apporté dans le milieu à une vitesse inférieure à la vitesse de consommation que la microalgue pourrait réaliser sans limitation.

Cela se traduit également par une absence de facteur nutritif résiduel dans le milieu de culture, la microalgue consommant ce facteur nutritif au fur et à mesure de son apport.

De préférence, le facteur nutritif est la source carbonée, et plus particulièrement le glucose.

La présente invention est ainsi particulièrement adaptée à la stabilisation des métabolites tels les caroténoïdes, dont l'extrême sensibilité à l'oxydation est bien connue de l'homme du métier.

Dans le cadre plus particulier de l'invention, la stabilisation des caroténoïdes garantit ainsi également celle des autres métabolites produits par les microalgues, métabolites susceptibles d'être dégradés par oxydation.

Plus particulièrement, les métabolites sensibles à l'oxydation sont stockés dans les cellules des microalgues contenues dans la biomasse.

Le procédé selon la présente invention rend inutile l'ajout d'antioxydant ou de stabilisant exogène pour préserver les métabolites sensibles à l'oxydation. Ainsi, de préférence, le procédé ne comprend pas l'ajout d'antioxydant ou de stabilisant exogène à la dite biomasse sèche.

La présente description décrit la mise à disposition une biomasse naturelle à teneur garantie en métabolites d'intérêt, comme les pigments.

La biomasse de microalgues s'entend ici de microalgues, plus particulièrement du genre *Chlorella,* tel que *Chlorella sorokiniana.* Dans un mode très particulier, la souche de *Chlorella sorokiniana* est la souche UTEX 1663 - *The Culture Collection of Algae at the University of Texas at Austin* - USA.

Les métabolites d'intérêt à stabiliser s'entendront plus particulièrement ici, comme il sera exemplifié ici, des caroténoïdes totaux, dont la lutéine, les Chlorophylles et les vitamines. Dans un mode préféré, le métabolite d'intérêt à stabiliser est la lutéine.

Pour la vitamine B12, non naturellement produite par la microalgue lorsque cultivée en hétérotrophie, elle est ajoutée au milieu de culture et assimilée par celle-ci.

Dans un mode de réalisation du procédé, la fermentation est réalisée dans des conditions particulières de culture hétérotrophique, qui garantissent une efficacité optimale quant à la stabilisation des métabolites d'intérêt sensibles à la dégradation oxydative produits par les microalgues.

Ainsi, le procédé comprend la fermentation d'une biomasse de microalgues en conditions hétérotrophiques avec une première étape de croissance de la biomasse et avec une deuxième étape de culture carencée en un facteur nutritif.

La production de biomasse comprend ainsi :
∘ une première étape de fermentation en mode « batch »,
∘ une deuxième étape de fermentation en mode « fed-batch » qui se traduit, lorsque la source carbonée est entièrement consommée par la microalgue, par l'apport en continu de ladite source carbonée à une vitesse inférieure à la vitesse de consommation que la microalgue pourrait réaliser sans limitation.

La société Demanderesse recommande de réaliser, en préambule de la phase de production de la biomasse proprement dite, une première phase de préculture (par fermentation en mode discontinu) qui permet alors la production d'une quantité de biomasse de microalgues nécessaire à l'ensemencement des fermenteurs de production proprement dits.

Par exemple, si l'on choisit de cultiver une souche de *Chlorella* telle *Chlorella sorokiniana,* on obtient au terme de cette première phase de préculture une densité cellulaire (classiquement mesurée par densité optique à 600 nm) d'une valeur comprise entre 50 et 60, comme il sera exemplifié ci-après.

Quant à la production de biomasse de microalgues, elle comprend donc une première étape de fermentation en mode « batch », avec un milieu de culture par exemple identique à celui mis en oeuvre à l'étape de préculture.

Lorsque la consommation de la source carbonée par la microalgue est totale (ici : glucose résiduel dans le milieu de culture = 0 g/l), on ajoute en continue ladite source carbonée à une vitesse inférieure à sa vitesse de consommation par la microalgue. Dans un mode préféré, le facteur nutritif carencé est le glucose.

La société Demanderesse a ainsi vaincu un préjugé technique, puisqu'il est communément admis que pour optimiser la production de lutéine par Chlorella (article de Wu et al cité *supra*), si la concentration élevée en glucose inhibe la croissance et la production de lutéine, il est recommandé de privilégier une concentration en glucose minimum, comprise entre 5 et 24 g/l dans le cas de la production de lutéine par C. *pyrenoidosa.*

Les conditions de culture préconisées selon l'invention ne sont donc pas compatibles avec celles communément admises dans l'état de l'art pour optimiser la production de lutéine.

Dans le cas de *Chlorella sorokiniana,* la société Demanderesse recommande d'ajouter du glucose à une vitesse supérieure à 1 g/l/h en maintenant le glucose résiduel à 0 g/l. Par exemple, la vitesse d'apport en glucose peut être comprise entre 1 et 5 g/l/h, plus particulièrement entre 2 et 4 g/l/h. Cette vitesse est choisie de sorte à ce que le glucose résiduel dans le milieu de culture soit à 0 g/l. Cette vitesse peut être définie au regard de la vitesse de consommation du glucose en absence de limitation. Ainsi, la vitesse d'apport en glucose peut être 90, 80, 70, 60 ou 50 % de cette vitesse de consommation du glucose en absence de limitation.

Cette vitesse se situe ainsi à environ 2 g/l/h en début de « fed batch », et elle peut augmenter jusqu'à 4 g/l/h en fin de culture.

La vitesse d'apport en facteur nutritif carencée est de préférence choisie de sorte à diminuer ou ralentir la vitesse de croissance cellulaire, tout en maintenant la croissance à une vitesse non-nulle. Notamment, il est proposé de diminuer la vitesse de croissance de 10 à 60 % par rapport à la vitesse de croissance sans limitation en glucose, notamment de 10, 15, 20, 25, 30, 35, 40, 45, 50 ou 55 % par rapport à la vitesse de croissance sans limitation en glucose. De préférence, la vitesse de croissance est diminuée de 15 à 55 %.

Par exemple, pour la souche de *Chlorella sorokiniana,* la Demanderesse recommande de choisir une vitesse d'apport en glucose permettant une croissance µ d'au moins 0,04 h⁻¹, par exemple comprise entre 0,06 h⁻¹ et 0,09 h⁻¹.

Ainsi, la vitesse d'apport en facteur nutritif est telle qu'elle permet une croissance cellulaire µ d'au moins 0,04 h⁻¹, par exemple comprise entre 0,06 h⁻¹ et 0,09 h⁻¹.

La durée de la phase de culture carencée en un facteur nutritif, notamment le glucose, est au moins 1 h, de préférence d'au moins 10 h, plus préférentiellement d'au moins 20 h, notamment entre 30 et 60 h.

La fermentation est arrêtée (apport de glucose stoppé) lorsque la quantité de biomasse souhaitée est atteinte (par exemple entre 30 et 80 g/l).

Une étude de stabilité (sur 14 mois et 23 mois) a été réalisée visant à étudier l'évolution de la teneur en caroténoïdes (lutéine), en vitamines (B9 et plus particulièrement B12) et en acides gras totaux de la biomasse obtenue par fermentation en conditions hétérotrophiques, en comparaison avec une biomasse produite en conditions phototrophiques.

Comme il sera exemplifié ci-après, on constate une bien meilleure stabilité des métabolites produits par la Chlorelle, lorsque la biomasse est préparée en conditions hétérotrophiques selon le procédé préféré de l'invention.

C'est ainsi que, par exemple, la biomasse obtenue en photobioréacteur a perdu pratiquement 80 % de sa concentration en lutéine après 14 mois de stockage, alors que celle de la biomasse préparée en conditions hétérotrophiques est inchangée.

La présente description décrit également l'utilisation d'une biomasse de microalgues produite en conditions hétérotrophiques comprenant une phase de culture carencée en un facteur nutritif pour stabiliser leurs métabolites sensibles à l'oxydation. De préférence, les microalgues sont choisies dans le groupe des microalgues du genre *Chlorella,* plus particulièrement *Chlorella sorokiniana. De préférence,* le facteur nutritif limitant est la source carbonée, en particulier le glucose.

La présente description décrit également une biomasse de microalgues du genre *Chlorella,* plus particulièrement *Chlorella sorokiniana,* obtenue par le procédé selon la présente invention, caractérisée en ce qu'elle contient au moins 1 g de lutéine par kg de biomasse après un stockage d'au moins 12, 18 ou 24 mois à température ambiante sans ajout d'antioxydant ou stabilisant exogène à la dite biomasse sèche.

L'invention sera mieux comprise à l'aide des exemples qui suivent.

### EXEMPLES

### Exemple 1. Préparation de la biomasse de C. sorokiniana cultivées en conditions hétérotrophiques selon le mode préféré de l'invention

### Phase 1. Préculture

La préculture permet la réactivation des souches et l'inoculation du fermenteur de production.

Elle est réalisée en Erlenmeyers à partir d'un tube congelé de souche de *Chlorella sorokiniana* (souche UTEX 1663 - *The Culture Collection of Algae at the University of Texas at Austin* - USA) et avec 600 ml de milieu de composition tel que présenté dans le tableau 1 ci-dessous :

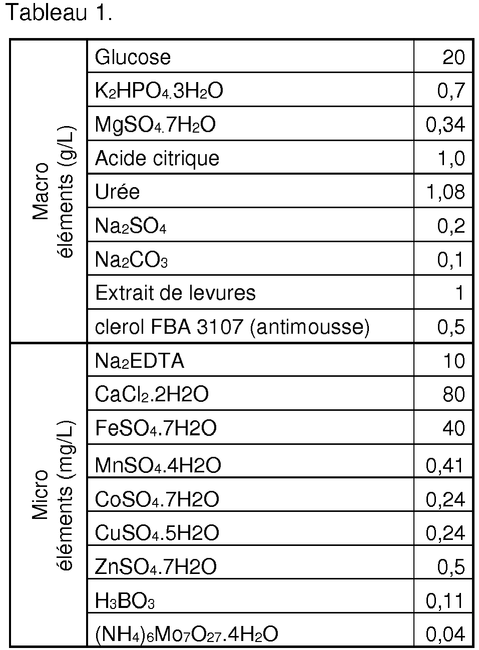

Le pH est ajusté à 7 avant stérilisation par ajout de NaOH 8N.

L'incubation se déroule à 28°C +/- 1°C sous agitation à 110 rpm (agitateur INFORS Multitron) pendant 72 heures.

La concentration finale en biomasse obtenue en fin d'incubation de chaque Erlenmeyer, par mesure de la DO à 600nm, se situe à environ 50-60.

### Phase 2. Production

Les paramètres de conduite de la fermentation sont présentés dans le tableau 2 suivant.

**Tableau 2.**

| | |
|---|---|
| Volume | 13.5 L Après Inoculation - 16 à 20 L en final |
| Température | 28 - 30 °C |
| pH | 6.5 - 6.6 par NH₃ 28% p/p |
| pO₂ | > 20% (maintenue par agitation) |
| Agitation | 300 rpm |
| Débit d'air | 15 L/min |

### Etape 1 : fermentation en mode « Batch »

Le milieu du batch est identique à celui de la préculture (tableau 1), mais sans l'extrait de levure et l'urée est remplacée par du NH₄Cl (tableau 3 ci-dessous).

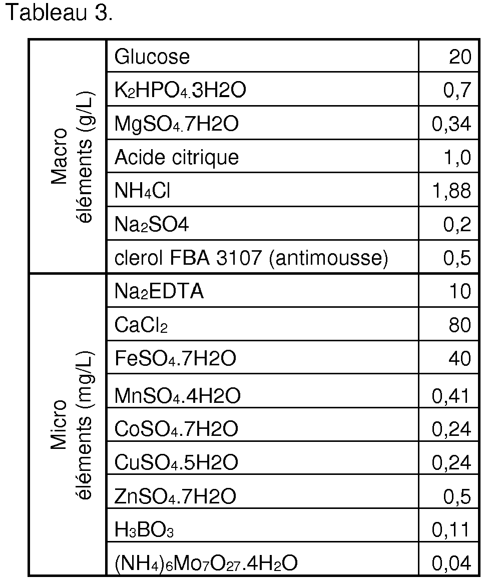

### Etape 2 : fermentation en mode « Fed-batch »

On ajoute également de la vitamine B12 en phase batch, à raison de 0,47 µg/l de milieu, de manière à ce qu'elle soit stockée dans la biomasse à une valeur de l'ordre de 400 µg / 100 g de biomasse sèche.

En phase de « Fed-batch », un apport de milieu complet est réalisé avec une alimentation en glucose en continu à une vitesse inférieure à la vitesse de consommation permise par la souche de sorte que la teneur résiduelle dans le milieu est nulle.

Cette vitesse d'apport en glucose se situe à environ 2 g/L_{Al}/h (Al = après inoculation) en début de « Fed-Batch » et elle peut augmenter jusque 4 g/L_{Al}/h en fin de culture. La croissance µ pendant cette étape est comprise entre 0,07 et 0,08 h⁻¹.

La concentration en glucose dans la solution d'alimentation peut aller de 400 à 800 g/L.

Les sels sont apportés en continu, séparément ou en mélange avec le glucose.

Le premier ajout doit être fait dès la fin du « batch », mais les sels ne doivent pas être apportés en une seule fois pour éviter d'inhiber la croissance de la souche.

Le tableau 4 ci-dessous donne les besoins en sels pour 100 g de glucose :

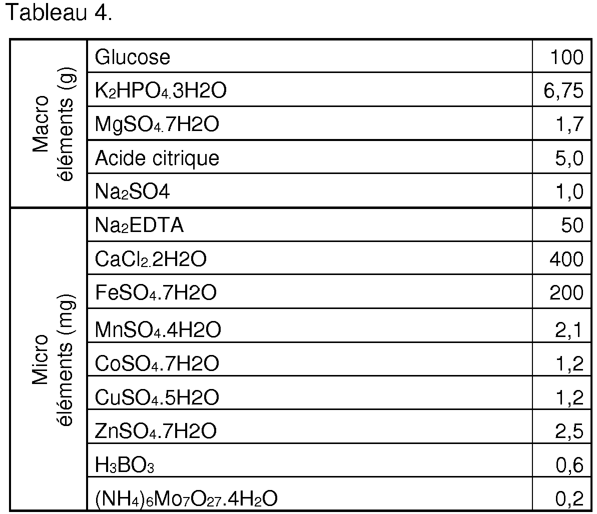

L'alimentation en glucose est stoppée lorsque la densité cellulaire atteint 80 g/l. Cela stoppe la culture.

La biomasse est atomisée à une matière sèche > 95 %.

### Exemple 2. Etudes de stabilité à température ambiante des métabolites sensibles à l'oxydation produits par Chlorella sorokiniana

Une étude de stabilité a été réalisée sur une période de 14 et de 23 mois à température ambiante, visant à étudier l'évolution de la teneur en caroténoïdes, en lutéine, en vitamine B9 et plus particulièrement la vitamine B12, extraits des biomasses obtenues en hétérotrophie (par fermentation) ou en autotrophie (photobioréacteurs).

Cette étude permet de démontrer l'impact du procédé de culture des chlorelles sur la stabilité de ces métabolites sensibles à l'oxydation.

Deux biomasses sont comparées :
- la première produite selon les conditions de l'exemple 1,
- la seconde produite en photobioréacteur (tel le PBR 4000), dans des conditions classiques (Pulz et al., 2000, in Rehm H.-J., Reed G. (Eds), Biotechnology, vol 10, Second edition, Weinheiom, 105-136).

Les mesures sont réalisées sur 100 g de biomasse à plus de 95 % MS (matière sèche), produites par ces deux conditions opératoires.

### Evolution de la teneur en caroténoïdes totaux et en lutéine (dosages réalisés par HPLC)

| | Biomasse produite par Fermentation | | | Biomasse produite en photobioréacteur | | |
|---|---|---|---|---|---|---|
| | t = 0 | t = 14 mois | t = 23 mois | t = 0 | t = 14 mois | t = 23 mois |
| Lutéine (mg / kg) | 2100 | 1950 | 1475 | 2650 | 525 | 375 |
| Caroténoïdes totaux (g / 100g) | 0,49 | 0,32 | 0,27 | 0,51 | 0,17 | 0,14 |

La stabilité des caroténoïdes totaux est plus élevée dans la biomasse obtenue en hétérotrophie.

Cette tendance est la même pour la lutéine ; la biomasse fermentée protège mieux la lutéine de la dégradation.

### Evolution de la teneur en vitamines (dosages réalisés selon méthodes AOAC 952.20 - Vitamine B12)

Apportée par le milieu de culture en hétérotrophie, la vitamine B12 a bien été assimilée par la biomasse de *Chlorella sorokiniana* ; ici son contenu est de l'ordre de 363 µg pour 100 g de biomasse sèche.

| | Biomasse produite par Fermentation | | | Biomasse produite en photobioréacteur | | |
|---|---|---|---|---|---|---|
| | t = 0 | t = 14 mois | t = 23 mois | t = 0 | t = 14 mois | t = 23 mois |
| Vitamine B12 (µg / 100 g) | 363 | 304 | nd | 120 | 107 | nd |

| | | | | | | |
|---|---|---|---|---|---|---|
| *Nd signifiant non-déterminé.* | | | | | | |

On observe une stabilité de la Vitamine B12 tout au long de l'étude dans les deux cas de figure. Les valeurs ont varié autour de 300µg/100g pour la biomasse produite en hétérotrophie et autour de 105µg/1400g pour la biomasse produite en autotrophie.

### Evolution de la teneur en Chlorophylles (dosages réalisés par spectrophotométrie)

| | Biomasse produite par Fermentation | | | Biomasse produite en photobioréacteur | | |
|---|---|---|---|---|---|---|
| | t = 0 | t = 14 mois | t = 23 mois | t = 0 | t = 14 mois | t = 23 mois |
| Chlorophylle A (g / 100 g) | 2,21 | 1,7 | 1,48 | 2,33 | 1,32 | 1,07 |
| Chlorophylle B (g / 100 g) | 0,55 | 0,51 | 0,5 | 0,57 | 0,57 | 0,53 |

Si la teneur en Chlorophylle B reste stable dans les deux conditions opératoires, ce n'est pas le cas pour celle en Chlorophylle A, bien mieux stabilisée dans la biomasse produite par fermentation.

En conclusion, les métabolites produits par les microalgues sont plus stables dans la biomasse produite par fermentation (ce qui traduit leur meilleure protection contre la dégradation oxydative à laquelle ils sont sensibles).

Ce phénomène est d'autant plus marqué pour la teneur en lutéine, remarquablement plus stable dans la biomasse fermentée (alors que dégradées à plus de 80 % dans la biomasse produite en autotrophie).

## Revendications

1. Procédé de stabilisation ou de stockage de métabolites sensibles à l'oxydation choisis dans le groupe constitué des caroténoïdes comme la lutéine, des acides gras monoinsaturés et polyinsaturés comme l'acide palmitoléique, l'acide oléique ou l'acide linoléique, des pigments chlorophylliens comme la chlorophylle A et B, et des vitamines comme les vitamines B9 et B12, pris seuls ou en combinaison, plus particulièrement des caroténoïdes, le dit procédé comprenant :
- la fermentation d'une biomasse de microalgues en conditions hétérotrophiques comprenant :
• Une première étape de fermentation en mode « batch »
• Une deuxième étape de fermentation en mode « fed batch » qui démarre, lorsque la source carbonée est entièrement consommée par la microalgue, par l'apport en continu de ladite source carbonée à une vitesse inférieure à sa vitesse de consommation par la microalgue ; et
- le stockage de la biomasse sèche au sein de laquelle les métabolites sensibles à l'oxydation sont stabilisés.

2. Procédé selon la revendication 1, **caractérisé en ce que** les microalgues sont choisies dans le groupe des microalgues du genre *Chlorella,* plus particulièrement *Chlorella sorokiniana.*

3. Procédé selon l'une ou l'autre des revendications 1 et 2, **caractérisé en ce que** le procédé ne comprend pas l'ajout d'antioxydant ou stabilisant exogène à la dite biomasse sèche.

4. Procédé selon l'une quelconque des revendications 1-3, **caractérisé en ce que** le facteur nutritif carencé est le glucose et qu'il est apporté à la culture à vitesse supérieure à 1 g/l/h, de préférence à une vitesse comprise entre 1 et 5 g/l/h, plus particulièrement entre 2 et 4 g/l/h.

5. Procédé selon l'une quelconque des revendications 1-4, **caractérisé en ce que** la durée de la phase de culture carencée est d'au moins 1 h, de préférence d'au moins 10 h, plus préférentiellement d'au moins 20 h, notamment entre 30 et 60 h.

6. Procédé selon l'une quelconque des revendications 1-5, **caractérisé en ce que** l'étape de stockage dure au moins 12, 18 ou 24 mois à température ambiante.

7. Procédé selon l'une quelconque des revendications 1-6, **caractérisé en ce que** le métabolite sensible à l'oxydation est la lutéine.

## Patentansprüche

1. Verfahren zur Stabilisierung oder Lagerung von für eine Oxidation empfindlichen Metaboliten, die gewählt sind aus der Gruppe, umfassend Carotenoide, wie Lutein, einfach und mehrfach ungesättigte Fettsäuren, wie Palmitoleinsäure, Oleinsäure, Linolsäure, Chlorophyllpigmente wie Chlorophyll A und B, und Vitamine wie die Vitamine B9 und B12, alleine oder in Kombination, insbesondere Carotenoide, wobei das Verfahren umfasst:
- die Fermentierung einer Biomasse aus Mikroalgen unter heterotrophischen Bedingungen, umfassend:
* einen ersten Fermentierungsschritt im "Batch"-Modus
* einen zweiten Fermentierungsschritt im "Fed Batch"-Modus, welcher beginnt, wenn die Kohlenstoffquelle vollständig von der Mikroalge verbraucht ist, durch die ständige Zufuhr der Kohlenstoffquelle in einer Geschwindigkeit, die geringer ist als die Verbrauchsgeschwindigkeit der Mikroalge; und
- die Lagerung der trockenen Biomasse, in welcher die für eine Oxidation empfindlichen Metaboliten stabilisiert sind.

2. Verfahren nach Anspruch 1, **dadurch gekennzeichnet, dass** die Mikroalgen gewählt sind aus der Gruppe der Mikroalgen der Gattung *Chlorella,* insbesondere *Chlorella sorokiniana.*

3. Verfahren nach Anspruch einem der Ansprüche 1 und 2, **dadurch gekennzeichnet, dass** das Verfahren nicht die Zugabe eines Antioxidans oder exogenen Stabilisators zur trockenen Biomasse umfasst.

4. Verfahren nach einem der Ansprüche 1-3, **dadurch gekennzeichnet, dass** der mangelnde Nährstofffaktor Glucose ist und, dass er der Kultur mit einer Geschwindigkeit größer 1 g/l/h, bevorzugt mit einer Geschwindigkeit von 1 bis 5 g/l/h, insbesondere mit einer Geschwindigkeit von 2 bis 4 g/l/h zugeführt wird.

5. Verfahren nach einem der Ansprüche 1-4, **dadurch gekennzeichnet, dass** die Dauer der Phase der Mangelkultur wenigstens 1 h, bevorzugt wenigstens 10 h, stärker bevorzugt wenigstens 20 h, insbesondere 30 bis 60 h beträgt.

6. Verfahren nach einem der Ansprüche 1-5, **dadurch gekennzeichnet, dass** der Schritt der Lagerung wenigstens 12, 18 oder 24 Monate bei Raumtemperatur dauert.

7. Verfahren nach einem der Ansprüche 1-6, **dadurch gekennzeichnet, dass** der für die Oxidation empfindliche Metabolit Lutein ist.

## Claims

1. A method for stabilizing or for storing oxidation-sensitive metabolites selected from the group consisting of carotenoids such as lutein, monounsaturated and polyunsaturated fatty acids, such as palmitoleic acid, oleic acid or linoleic acid, chlorophyll pigments such as chlorophyll A and B, and vitamins such as vitamins B9 and B12, alone or in combination, more particularly carotenoids, said method comprising:
- fermenting a biomass of microalgae under heterotrophic conditions comprising:
• A first fermentation step in «batch» mode
• A second fermentation step in «fed-batch» mode which starts, when the carbon-containing source is completely consumed by the microalgae, by continuously supplying said carbon-containing source at a rate lower than its rate of consumption by the microalgae; and
- storing the dry biomass in which the oxidation-sensitive metabolites are stabilized.

2. Method according to claim 1, wherein the microalgae are selected from the group of the genus *Chlorella,* more particularly *Chlorella sorokiniana.*

3. Method according to either of claims 1 and 2, wherein the method does not comprise adding exogenous antioxidant or stabilizer to said dry biomass.

4. Method according to any one of claims 1 to 3, wherein the deficient nutrient factor is glucose and it is supplied to the culture phase at a rate above 1 g/l/h, preferably at a rate between 1 and 5 g/l/h and more preferably at a rate between 2 and 4 g/l/h.

5. Method according to any one of claims 1 to 4, wherein the duration of the deficient culture phase is at least 1 h, preferably at least 10 h, and more preferably at least 20 h, especially between 30 and 60 h.

6. Method according to any one of claims 1 to 5, wherein the storage step lasts at least 12, 18 or 24 months at room temperature.

7. Method according to any one of claims 1 to 6, wherein the oxidation-sensitive metabolite is lutein.
